# EUROPEAN PATENT APPLICATION

(11) **EP 3 936 125 A1**
(43) Date of publication of application: **12.01.2022**
(21) Application number: 20787426.4
(22) Date of filing: 08.04.2020
(51) Int. Cl.: A61K 31/365, A61K 9/00, A61P 25/02, A61P 25/00

(54) **USE OF GINKGO TERPENE LACTONE IN PREPARING DRUG FOR PREVENTING AND/OR TREATING GUILLAIN-BARRÉ-STROHL SYNDROME**

(30) Priority: 10.04.2019 CN 201910286519; 25.11.2019 CN 201911167420
(71) Applicant: Chengdu Baiyu Pharmaceutical Co., Ltd., Chengdu, Sichuan 611130 (CN)
(72) Inventor: LI, Huiqin, Chengdu, Sichuan 611130 (CN); TANG, Yongxin, Chengdu, Sichuan 611130 (CN); LIU, Ke, Chengdu, Sichuan 611130 (CN)
(74) Representative: J A Kemp LLP
(86) International application number: PCT/CN2020/083678
(87) International publication number: WO 2020/207398

(57) **Abstract**

A use of ginkgo terpene lactone in preparing a drug for preventing and/or treating Guillain-Barre-Strohl syndrome. Ginkgo terpene lactone has certain effects on relieving the condition of EAN mice, and the use of a combination of different ginkgo terpene lactone monomer compounds shows a certain synergistic effect. Ginkgo terpene lactone can be used clinically to prevent and/or treat the symptoms of Guillain-Barre-Strohl syndrome, and no adverse reactions have been observed. The means of obtaining the medical raw materials is common, preparation costs are lower, the costs of patient treatment are low, and safety is high.

## Description

### TECHNICAL FIELD

The present disclosure relates to the technical field of medicine, and in particular relates to a use of ginkgo terpene lactone in the manufacture of a medicament for preventing and/or treating Guillain-Barre-Strohl syndrome.

### BACKGROUD

Guillain-Barre-Strohl syndrome (GBS) is an immunologically mediated disease that mainly affects a peripheral nervous system. Currently, it is considered that, Guillain-Barre-Strohl syndrome may be triggered by various infection and non-infection factors and is one of common reasons for acute quadriplegia at present.

Currently, it is considered that, GBS mainly refers to abnormal immune responses triggered by infectious pathogens, involving cellular immunity and humoral immunity. Cellular immunity: circulating T cells are activated by unknown antigens, and this process is implemented by antigen presenting cells (APCs) by using a major histocompatibility complex (MHC), a T cell receptor (TCR) and a co-stimulus signal; these activated neurogenic T cells are proliferated and differentiated into Th1, Th2 and Th17, and secreted immune inflammatory factors reach a peripheral nervous system via a damaged blood-nerve barrier (BNB); Th1 mainly secrets TNF-α and IFN-γ to activate macrophages; macrophages secret NO, MMP and TNF-α, so as to damage myelin sheaths and BNB and further promote proliferation of Th1; Th2 mainly secrets IL-4 and IL-6, so as to promote activation of B lymphocytes and cause humoral immunity; and Th17 mainly secrets IL-17 and IL-22, so as to aggravate damage to BNB and peripheral neuritis. Humoral immunity: B lymphocytes are activated by unknown antigens through APCs and converted into plasmocytes to secret antibodies, and the activated B lymphocytes and the antibodies reach a peripheral nervous system via damaged BNB; and these antibodies can activate macrophages and induce antibody-dependent cell-mediated cytotoxicity (ADCC) and can further activate a complement system to form a membrane attack complex, to finally cause damage to axons and myelin sheaths.

Early in 1985, clinical research has proved the effect of PE (plasma exchange) on GBS treatment, and especially PE treatment may accelerate rehabilitation of a patient who was unable to walk within 2 weeks after attack. In 1992, random experiments have proved that IVIG (intravenous immunogloblin G) is effective in GBS treatment. In 1993, glucocorticoids had no effect on GBS treatment. In 1997, IVIG plus PE were not superior to single IVIG in GBS treatment. In 2004, IVIG plug hormone were not superior to single IVIG in GBS treatment. In 2016, New Zealand SID-GBS studied whether GBS with poor prognosis may benefit after IVIG was applied again. Likewise, this content is also included in multi-center study of The International Guillain-Barre Syndrome Outcome Study, and results are expected to be released. In 2017, a Phase 2 multi-center random clinical test of a small-scale clinical test on GBS treatment of a complement inhibitor (eculizumab) was started, and research results are expected. Currently, PE and IVIG are recognized as effective GBS immune therapies. PE may remove macromolecule particles in plasma, including cytokines, immune complexes and other inflammatory substances, but PE may cause blood pressure change and arrhythmia, use of a central catheter and hemorrhage, and may be accompanied by septicemia. IVIG may make some patients recover from an acute phase and reduce a death rate, but intravenous administration of gamma globulin also causes various side effects. Through long-term follow-up, it is found that, some patients still suffer from dysneuria to different extents. Therefore, further exploration of a novel method for GBS treatment is still an urgent problem to be solved in neurology.

Ginkgo terpene lactone is a medicinal composition in gingko leaf extracts. Ginkgo terpene lactone has functions of resisting allergies, resisting inflammation, resisting shock, preventing ischemic injuries, preventing organ transplant rejections, and so on (GUAN Xiaoju, Advances in Studies on Pharmacological Activities of Ginkgolides, Vol.22, No.3, 1995). XU Jiangping et al. have reported that ginkgolides may reduce resistance to cerebral vessels of anesthetized dogs and increase cerebral blood flow, but do not affect the heart rate and blood pressure (XU Jiangping et al., Effects of Ginkgolide on Cerebral Blood Flow in Dogs, Journal of Chinese Integrative Medicine, 2005-01-15). Currently, there is no report that ginkgo terpene lactones or ginkgo extracts are used to prevent and/or treat Guillain-Barre-Strohl syndrome.

### SUMMARY

The present disclosure provides a novel use of ginkgo terpene lactone, i.e., a use of ginkgo terpene lactone in the manufacture of a medicament for preventing and/or treating Guillain-Barre-Strohl syndrome.

Disclosed is a use of ginkgo terpene lactone or pharmaceutically-acceptable salts, esters, hydrates, solvates and isomers thereof, or any crystal form, racemate and metabolin thereof, or a mixture thereof in the manufacture of a medicament for preventing and/or treating Guillain-Barre-Strohl syndrome. As shown in test example 1 of the present disclosure, the inventors explored, by studying scores of neurological symptoms of EAN (experimental autoimmune neuritis) model rats before and after medication of ginkgo terpene lactone, whether ginkgo terpene lactone may treat Guillain-Barre-Strohl syndrome. An EAN model is currently recognized as an ideal animal model of Guillain-Barre-Strohl syndrome in the medical field, and the model has significance in studies in clinical neuroimmunology. From test example 1, it can be learned that, ginkgo terpene lactone can remarkably reduce scores of clinical symptoms of rats and remarkably improve an EAN epidemic situation of rats.

Preferably, the ginkgo terpene lactone is ginkgo sesquiterpene lactone or/and ginkgo diterpene lactone.

Preferably, the ginkgo terpene lactone is one or two or more of ginkgolide A, ginkgolide B, ginkgolide C, ginkgolide M, ginkgolide J, ginkgolide K, ginkgolide L, ginkgolide N, ginkgolide P, ginkgolide Q and bilobalide.

Preferably, the ginkgo terpene lactone is one combination selected from bilobalide:ginkgolide B=(35-60):(40-65) w/w; or ginkgolide A:ginkgolide C=(40-75):(25-60) w/w; or ginkgolide M:ginkgolide K=(30-60):(40-70) w/w; or ginkgolide A:ginkgolide B:ginkgolide C=1:1:1 (w/w/w); or ginkgolide A:ginkgolide B:ginkgolide C:bilobalide=12:34:6:48 (w/w/w/w).

Preferably, the ginkgo terpene lactone is a combination of ginkgolide A:ginkgolide B:ginkgolide C:bilobalide=(10-35):(20-38):(5-14):(26-50) w/w/w/w.

Preferably, the ginkgo terpene lactone is a combination selected from bilobalide:ginkgolide B=50:50 (w/w); or ginkgolide A:ginkgolide C=50:50 (w/w); or ginkgolide M:ginkgolide K=50:50 (w/w).

Through research, the inventors found that, a ginkgo terpene lactone monomer compound or various ginkgo terpene lactone compounds in combined use may reduce scores of neurological symptoms of rats and improve an epidemic situation of EAN rats. Moreover, for various ginkgo terpene lactone compounds in combined use, the inventors also found that, medicines in combined use in the above proportion may treat the epidemic situation of EAN rats, and may further acquire a therapeutic effect superior than that of any original ginkgo terpene lactone compound in single use, to have a synergistic effect.

Preferably, disease subtypes of the Guillain-Barre-Strohl syndrome include acute inflammatory demyelinating polyneuropathy, acute motor axonal neuropathy, acute motor sensory axonal neuropathy, Miller Fisher syndrome, acute panautonomic neuropathy and acute sensory neuropathy.

The present disclosure further provides a medicament for preventing and/or treating Guillain-Barre-Strohl syndrome. The medicament includes ginkgo terpene lactone as an active ingredient, and a pharmaceutically-acceptable carrier. The ginkgo terpene lactone is one combination selected from bilobalide:ginkgolide B=(35-60):(40-65) w/w; or ginkgolide A:ginkgolide C=(40-75):(25-60) w/w; or ginkgolide M:ginkgolide K=(30-60):(40-70) w/w; or ginkgolide A:ginkgolide B:ginkgolide C=1:1:1 (w/w/w); or ginkgolide A:ginkgolide B:ginkgolide C:bilobalide=12:34:6:48 (w/w/w/w).

Preferably, the carrier includes one or more selected from a filler, a diluent, a lubricant, a flow aid, an anti-adherent, a dispersing agent, a wetting agent, a binder, a modifier, a solubilizer, an antioxidant, a bacteriostat, an emulsifier and a disintegrant; wherein the binder includes one or more selected from Arabic gum, gelatin, sorbitol, tragacanth gum, cellulose, microcrystalline cellulose, sodium carboxymethyl cellulose, ethyl cellulose, hydroxy propyl methyl cellulose, syrup, starch slurry and polyvinylpyrrolidone; wherein the filler includes one or more selected from lactose, powdered sugar, dextrin, starch and a derivative thereof, cellulose and a derivative thereof, an inorganic calcium salt, sorbitol and glycine; wherein the lubricant includes one or more selected from micronized silica gel, magnesium stearate, talcum powder, aluminum hydroxide, boric acid, hydrogenated vegetable oil and polyethylene glycol; wherein the disintegrant includes one or more selected from starch and a derivative thereof, polyvinylpyrrolidone and microcrystalline cellulose; wherein the wetting agent includes one or more selected from sodium lauryl sulfate, water and alcohol; wherein the antioxidant includes one or more selected from sodium sulfite, sodium hydrogen sulfite, sodium metabisulfite and dibutyl benzoic acid; wherein the modifier includes one or more selected from hydrochloric acid, citric acid, potassium hydroxide, sodium citrate and a buffer agent; wherein the emulsifier includes one or more selected from polysorbate-80, fatty acid sorbitan, Pluronic F-68, lecithin and soybean lecithin; and wherein the solubilizer includes one or more selected from Tween-80, bile and glycerin.

The present disclosure further provides a preparation containing the foregoing medicament. The preparation is a tablet, a capsule, a granule, a pill, an injection, an injection or a powder injection.

The present disclosure further provides a method for the preparation of a medicament for preventing and/or treating Guillain-Barre-Strohl syndrome. The method includes mixing an effective amount of ginkgo terpene lactone and a medicine carrier.

The present disclosure further provides a method for preventing and/or treating Guillain-Barre-Strohl syndrome. The method includes administering a therapeutically effective amount of the ginkgo terpene lactone to a subject.

The ginkgo terpene lactone disclosed by the present disclosure has an effect of relieving conditions of EAN mice, and the use of a combination of different ginkgo terpene lactone monomer compounds shows a synergetic effect.

Compared with gamma globulin, the ginkgo terpene lactone for treating Guillain-Barre-Strohl syndrome provided by the present disclosure has the advantage of being easier to obtain. Gamma globulin is extracted from plasma of healthy people, but the ginkgo terpene lactone is extracted from gingko leaves and purified, so that a medicine source is common, preparation costs are lower, and the ginkgo terpene lactone has broad application prospects.

### DETAILED DESCRIPTION

As described in the present disclosure, the term "preventing" means preventing occurrence of a disease and/or preventing relapse of a disease.

As described in the present disclosure, the term "Guillain-Barré-Strohl syndrome" is equivalent to "Guillain-Barre syndrome", equivalent to "Guillain-Barre syndrome", and equivalent to "Barre-guillain syndrome".

As described in the present disclosure, a synergistic effect means that two medicines in combined use, having an effect greater than that of medicines in single use, are deemed to have a synergistic effect.

Guillain-Barre-Strohl syndrome described in the present disclosure includes following disease subtypes: acute inflammatory demyelinating polyneuropathy (AIDP), acute motor axonal neuropathy (AMAN), acute motor sensory axonal neuropathy (AMSAN), Miller Fisher syndrome (MFS), acute panautonomic neuropathy and acute sensory neuropathy (ASN).

### Test example 1 Treatment effect of ginkgo terpene lactone on experimental autoimmune neuritis (EAN) model rats

### 1. Materials and methods

### 1.1 Experimental animals

200 female 42-48-day-old SPF (specific pathogen free) Lewis rats, purchased from Beijing Vital River Laboratory Animal Technology Co., Ltd., Animal Production License No.: SCXK(Jing)2016-0011, and Animal Certificate No.: No. 11400700299780.

### 1.2 Reagents and medicines

Peripheral myelin antigenic peptide P2 53-78, customized and synthesized by entrusting GL Biochem (Shanghai) Ltd. (Specific peptide structure: THR GLU SER PRO PHE LYS ASN THR GLU ILE SER PHE LYS LEU GLY GLN GLU PHE GLU GLU THR THR ALA ASP ASN ARG). Incomplete Freund's adjuvant, Sigma-Aldrich, Lot#SLBQ2284V Mycobacterium Tuberculosis, H37Ra, heat-inactived mycobacterium tuberculosis dry powder, BD Difco.

### 1.3 Grouping and Modeling

Persons skilled in the art learn that, pharmacological effect and dose are in a proportional relationship, i.e., a dose-effect relationship, only within a certain range. The applicant of the present disclosure has already completed relevant discovery of a dose-effect relationship of ginkgo terpene lactone and EAN model mice through preliminary experiments in an early stage, finding that when a total administration dosage of ginkgo terpene lactone is within a range of 0-4.5 mg/kg, a medicine effect presents a continuously increasing quantitative change along with a medicine dosage, and when the total administration dosage of ginkgo terpene lactone is within a range of 4.5-15 mg/kg, the medicine effect, at the maximum value, does not present a continuous quantitative change along with the medicine dosage. Detailed are shown in Table 1. Therefore, in Test example 1 of the present disclosure, when the administration dosage is set within a range of 4.5-15 mg/kg, the impact of the dosage-effect relationship of the medicine on research results may be eliminated.
20 experimental groups, 10 mice in each group.
Negative control group: normal saline
Model control group: normal saline
Ginkgolide A group (GA group): 4.5 mg/kg
Ginkgolide B group (GB group): 4.5 mg/kg
Ginkgolide C group (GC group): 4.5 mg/kg
Ginkgolide J group (GJ group): 4.5 mg/kg
Ginkgolide M group (GM group): 4.5 mg/kg
Ginkgolide K group (GK group): 4.5 mg/kg
Bilobalide group (BB group): 4.5 mg/kg
Ginkgolide I group (GA:GC=40:60): 8.0 mg/kg
Ginkgolide II group (GA:GC=75:25): 6.0 mg/kg
Ginkgolide III group (GA:GC=50:50): 9 mg/kg
Ginkgolide IV group (GM:GK=30:70): 6.4 mg/kg
Ginkgolide V group (GM:GK=60:40): 7.5 mg/kg
Ginkgolide VI group (GM:GK=50:50): 9 mg/kg
Ginkgolide VII group (GB:BB=40:60): 7.5 mg/kg
Ginkgolide VIII group (GB:BB=65:35): 6.9 mg/kg
Ginkgolide IX group (GB:BB=50:50): 9 mg/kg
Ginkgolide X group (GA:GB:GC=1:1:1): 13.5 mg/kg
Ginkgolide XI group (GA:GB:GC:BB=12:34:6:48): 9.4 mg/kg
Ratios of the ginkgo terpene lactone monomer compounds in combined use are all mass ratios.

The principle of setting mass ratios of the ginkgo terpene lactone monomer compounds in combined use in pairs is that: the inventors first make a test on two ginkgo terpene lactone monomer compounds in combined use based on a mass ratio of 1:1, to explore a difference between the effect of the two medicines in combined use and the effect of the medicines in single use, and the two medicines in combined use have a synergistic effect when having an effect greater than that of medicines in single use.

When the two medicines in combined use have a synergistic effect, the inventors further explore the mass ratio of two ginkgo terpene lactone monomer compounds, to study the optimal range of the mass ratio at which two medicines in combined use have a synergistic effect.

The inventors further explore whether three and four ginkgo terpene lactone monomer compounds in combined use have a synergistic effect.

Intragastric administration, 2 times a day. Medicines were dissolved in accessories (glycerin: ethanol=6:4 V/V) to reach 5 mg/ml, and then were configured to reach corresponding concentrations by using sterilized drinking water.

For each rat in an EAN model group, an inoculum was subcutaneously injected into foot pads of hind limbs on two sides, 200 µL/rat (100 µL per side). The inoculum for each rat contains 200 µg of antigenic peptide P2 53-78, 100 µL of incomplete Freund's adjuvant, 2 mg of H37Ra, and 100 µL of 0.9% sodium chloride injection. For the negative control group, isopyknic normal saline was injected. For each group of animals, medicine administration was started from the first day after immunization, for 30 days in total.

### 1.4 Scores of neurological symptoms and changes of body weight

The day of molding serves as the 0th day, and after that is 30 days of observation period. Observation date is represented by D plus number, D1, D2 and D3 respectively represent Observation date 1, Observation date 2 and Observation date 3, and subsequent dates are represented in the same way. Rats were weighed once every two days, and clinical symptoms of all experimental animals were scored. Within one week of a peak period (D14-D20) of disease attack of animals, weighing and clinical symptom scoring were performed every day. Score standards are as below: 0, normal; 1, a soft tail; 2, unable to lift a tail; 3, unable to automatically correct a body position; 4, ataxia; 5, mild paralysis of hind limbs; 6, moderate paralysis of hind limbs; 7, severe paralysis of hind limbs; 8, paresis of four limbs; 9, severe paresis of four limbs; and 10, death.

### 1.5 Test on algesia of ankle joint

Near a peak period of disease attack of EAN model animals, a test on algesia of ankle joints was performed on EAN model animals and adjuvant control animals, and rats in a rest state were forced to bend hind legs towards foot soles (dorsiflexion). For each side, 5 tests were made, at an interval of at least 5 s. For each time of dorsiflexion, if rats screamed or obviously withdrew paws, 1 score was counted. For each side, a total score is 0-5.

### 1.6 Mathematical statistics

Data is represented by mean±standard deviation ( x±SD). For scores of symptoms and scores of algesia, a non-parametric test Mann-Whitney U was used to compare differences between two groups. When P is less than 0.05, a statistic difference occurs.

### 2. Experimental results

**Table 1 Impact of different dosages of ginkgo terpene lactone on neurological symptoms of EAN model rats (Pre-experimental study-D17)**

| Group | | Clinical score (D17) | |
|---|---|---|---|
| | 2.0 mg/kg | 4.5 mg/kg | 15 mg/kg |
| Negative control | 0.0±0.0^{∗} | -- | -- |
| Model control | 7.90±0.99 | -- | -- |
| Ginkgolide A group | 7.00±0.82^{∗} | 6.10±0.74^{∗△} | 6.00±1.25^{∗△} |
| Ginkgolide B group | 6.10±1.10^{∗} | 5.10±0.57^{∗△} | 5.20±0.63^{∗△} |
| Ginkgolide C group | 6.90±0.88^{∗} | 6.10±0.57^{∗△} | 6.00±0.67^{∗△} |
| Ginkgolide J group | 7.10±0.57^{∗△} | 6.40±0.84^{∗△} | 6.30±0.82^{∗△} |
| Ginkgolide M group | 7.10±0.32^{∗} | 6.40±0.97^{∗△} | 6.50±0.71^{∗△} |
| Ginkgolide K group | 7.20±0.42^{∗} | 6.50±0.85^{∗△} | 6.70±0.82^{∗△} |
| Bilobalide group | 5.70±0.67^{∗} | 4.80±0.63^{∗△} | 4.90±0.88^{∗△} |

| | | | |
|---|---|---|---|
| Note: compared with the model control group, ^{∗}P is less than 0.05; and compared with a low dosage of a same product, ^{△}P is less than 0.05. | | | |

**Table 2 Impact of ginkgo terpene lactone on neurological symptoms of EAN model rats**

| Group | Clinical score | | | | | | | | | |
|---|---|---|---|---|---|---|---|---|---|---|
| | D12 | D14 | D16 | D17 | D18 | D19 | D20 | D22 | D24 | D26 |
| Negative control | 0.0±0.0^{∗} | 0.0±0.0^{∗} | 0.0±0.0^{∗} | 0.0±0.0^{∗} | 0.0±0.0^{∗} | 0.0±0.0^{∗} | | 0.0±0.0^{∗}0.0±0.0^{∗} | 0.0±0.0^{∗} | 0.0±0.0^{∗} |
| Model control | 4.5±0.8 | 5.7±0.7 | 7.3±0.7 | 8.1±0.7 | 7.7±0.5 | 6.8±0.4 | 6.1±0.7 | 4.9±0.7 | 4.2±0.6 | 3.5±0.5 |
| Ginkgolide A group | 4.4±0.5 | 5.3±0.5 | 6.0±0.5^{∗} | 6.7±0.7^{∗} | 6.1±0.6^{∗} | 5.5±0.5^{∗} | 5.1±0.6^{∗} | 4.4±0.5 | 3.7±0.8 | 3.1±0.6 |
| Ginkgolide B group | 3.4±0.5^{∗} | 3.8±0.6^{∗} | 4.5±0.5^{∗} | 5.1±0.6^{∗} | 4.8±0.4^{∗} | 4.2±0.4^{∗} | | 3.9±0.6^{∗}3.3±0.5^{∗} | 2.9±0.7^{∗} | 2.4±0.5^{∗} |
| Ginkgolide C group | 4.4±0.7 | 5.2±0.8 | 6.0±0.7^{∗} | 6.8±0.8^{∗} | 6.1±0.6^{∗} | 5.5±0.5^{∗} | 5.1±0.6^{∗} | 4.4±0.5 | 3.8±0.8 | 3.1±0.6 |
| Ginkgolide J group | 4.4±0.7 | 5.5±0.5 | 6.5±0.5^{∗} | 7.4±0.5^{∗} | v.9±0.6^{∗} | 6.2±0.6^{∗} | 5.6±0.5 | 4.6±0.5 | 4.0±0.7 | 3.2±0.6 |
| Ginkgolide M group | 4.5±0.7 | 5.6±0.5 | 6.4±0.5^{∗} | 7.3±0.5^{∗} | 6.8±0.6^{∗} | 6.1±0.6^{∗} | 5.5±0.5 | 4.6±0.5 | 4.0±0.7 | 3.2±0.6 |
| Ginkgolide K group | 4.5±0.7 | 5.6±0.5 | 6.4±0.5^{∗} | 7.2±0.4^{∗} | 6.7±0.5^{∗} | 6.0±0.5^{∗} | 5.5±0.5 | 4.5±0.5 | 3.9±0.7 | 3.1±0.6 |
| Bilobalide group | 3.6±0.5^{∗} | 3.9±0.6^{∗} | 4.6±0.5^{∗} | 5.2±0.6^{∗} | 4.9±0.6^{∗} | 4.3±0.5^{∗} | | 4.0±0.7^{∗}3.4±0.5^{∗} | 2.9±0.7^{∗} | 2.4±0.5^{∗} |
| Ginkgolide I group (A:C=40:60) | 3.9±0.6 | 4.4±0.5^{∗} | 5.3±0.5^{∗} | 6.1±0.6^{∗} | 5.4±0.7^{∗} | 5.0±0.5^{∗} | | 4.4±0.7^{∗}3.9±0.6^{∗} | 3.3±0.5^{∗} | 2.7±0.7^{∗} |
| Ginkgolide II group (A:C=75:25) | 4.0±0.5 | 4.5±0.5^{∗} | 5.2±0.4^{∗} | 6.1±0.6^{∗} | 5.5±0.5^{∗} | 5.0±0.0^{∗} | | 4.4±0.5^{∗}4.0±0.5^{∗} | 3.2±0.6^{∗} | 2.6±0.7^{∗} |
| Ginkgolide III group (A:C=50:50) | 3.7±0.5^{∗} | 4.1±0.6^{∗} | 4.7±0.5^{∗} | 5.5±0.5^{∗} | 4.9±0.6^{∗} | 4.5±0.5^{∗} | | 4.1±0.7^{∗}3.6±0.5^{∗} | 3.0±0.8^{∗} | 2.6±0.7^{∗} |
| Ginkgolide IV group (M:K=30:70) | 4.4±0.5 | 5.3±0.5 | 6.0±0.0^{∗} | 6.8±0.4^{∗} | 6.2±0.4^{∗} | 5.5±0.5^{∗} | 5.2±0.4^{∗} | 4.4±0.5 | 3.7±0.8 | 3.1±0.6 |
| Ginkgolide V group (M:K=60:40) | 4.5±0.5 | 5.4±0.5 | 5.9±0.3^{∗} | 6.7±0.5^{∗} | 6.1±0.6^{∗} | 5.4±0.5^{∗} | 5.1±0.6^{∗} | 4.4±0.5 | 3.8±0.6 | 3.0±0.7 |
| Ginkgolide VI group (M:K=50:50) | 4.0±0.7 | 4.8±0.4^{∗} | 5.4±0.5^{∗} | 6.2±0.4^{∗} | 5.5±0.5^{∗} | 4.7±0.5^{∗} | | 4.5±0.5^{∗}4.1±0.6^{∗} | 3.6±0.7 | 3.0±0.7 |
| Ginkgolide VII group (GB:BB=40:60) | 2.9±0.3^{∗} ) | 3.2±0.4^{∗} | 3.9±0.3^{∗} | 4.3±0.5^{∗} | 4.2±0.4^{∗} | 3.6±0.5^{∗} | | 3.3±0.5^{∗}2.9±0.3^{∗} | 2.4±0.5^{∗} | 2.1±0.3^{∗} |
| Ginkgolide VIII group (GB:BB=65:35) | 2.8±0.4^{∗} ) | 3.1±0.3^{∗} | 3.8±0.4^{∗} | 4.1±0.6^{∗} | 4.0±0.5^{∗} | 3.5±0.5^{∗} | | 3.1±0.6^{∗}2.8±0.4^{∗} | 2.3±0.5^{∗} | 1.9±0.3^{∗} |
| Ginkgolide IX group | 2.7±0.5^{∗} | 3.0±0.0^{∗} | 3.3±0.5^{∗} | 3.5±0.5^{∗} | 3.5±0.5^{∗} | 2.9±0.3^{∗} | | 2.8±0.4^{∗}2.7±0.5^{∗} | 2.3±0.5^{∗} | 1.9±0.3^{∗} |
| (GB:BB=50:50) | | | | | | | | | | |
| Ginkgolide X group (GA:GB:GC =1:1:1) | 3.1±0.6^{∗} | 3.4±0.5^{∗} | 4.1±0.6^{∗} | 4.5±0.5^{∗} | 4.3±0.5^{∗} | 3.7±0.5^{∗} | 3.3±0.5^{∗}2.9±0.3^{∗} | | 2.4±0.5^{∗} | 2.1±0.3^{∗} |
| Ginkgolide XI group (A:B:C:BB =12:34:6:48) | 2.5±0.5^{∗} | 3.0±0.0^{∗} | 3.4±0.5^{∗} | 3.6±0.7^{∗} | 3.5±0.5^{∗} | 3.0±0.5^{∗} | 2.6±0.5^{∗}2.5±0.5^{∗} | | 2.2±0.4^{∗} | 1.7±0.5^{∗} |

| | | | | | | | | | | |
|---|---|---|---|---|---|---|---|---|---|---|
| Note: compared with the model control group, ^{∗}P is less than 0.05. | | | | | | | | | | |

Results are shown in Table 2. In this test example, compared with the model group, the ginkgo terpene lactone monomer compounds in single use and in combined use by twos, threes and fours may reduce clinical cores within different time ranges, and differences have statistical significance. This shows that the foregoing medicine administration groups may mitigate clinical symptoms of EAN rats.

**Table 3 Synergistic effect of ginkgolide A and ginkgolide C in combined use**

| Group | Clinical score | | | | | | | |
|---|---|---|---|---|---|---|---|---|
| | D12 | D14 | D16 | D17 | D18 | D19 | D20 | D22 |
| Ginkgolide A group | 4.4±0.5 | 5.3±0.5 | 6.0±0.5 | 6.7±0.7 | 6.1±0.6 | 5.5±0.5 | 5.1±0.6 | 4.4±0.5 |
| Ginkgolide C group | 4.4±0.7 | 5.2±0.8 | 6.0±0.7 | 6.8±0.8 | 6.1±0.6 | 5.5±0.5 | 5.1±0.6 | 4.4±0.5 |
| Ginkgolide I group (A:C=40:60) | 3.9±0.6 | 4.4±0.5# | 5.3±0.5# | 6.1±0.6# | 5.4±0.7# | 5.0±0.5# | 4.4±0.7# | 3.9±0.6 |
| Ginkgolide II group (A:C=75:25) | 4.0±0.5 | 4.5±0.5# | 5.2±0.4# | 6.1±0.6# | 5.5±0.5# | 5.0±0.0# | 4.4±0.5# | 4.0±0.5 |
| Ginkgolide III group (A:C=50:50) | 3.7±0.5# | 4.1±0.6# | 4.7±0.5# | 5.5±0.5# | 4.9±0.6# | 4.5±0.5# | 4.1±0.7# | 3.6±0.5# |

| | | | | | | | | |
|---|---|---|---|---|---|---|---|---|
| Note: compared with the single-use group (GA group) with a better effect, #P is less than 0.05. | | | | | | | | |

As shown in Table 3, in this test example, compared with the GA group with a better effect, the ginkgolide I group and the ginkgolide II group have lower clinical scores in D14-D20, the ginkgolide III group has lower clinical scores in D12-D22, and differences have statistical significance. This shows that ginkgolide A and ginkgolide C in combined use have a synergistic effect.

**Table 4 Synergistic effect of ginkgolide M and ginkgolide K in combined use**

| Group | Clinical score | | | | | |
|---|---|---|---|---|---|---|
| | D14 | D16 | D17 | D18 | D19 | D20 |
| Ginkgolide M group | 5.6±0.5 | 6.4±0.5 | 7.3±0.5 | 6.8±0.6 | 6.1±0.6 | 5.5±0.5 |
| Ginkgolide K group | 5.6±0.5 | 6.4±0.5 | 7.2±0.4 | 6.7±0.5 | 6.0±0.5 | 5.5±0.5 |
| Ginkgolide IV group | 5.3±0.5 | 6.0±0.0# | 6.8±0.4# | 6.2±0.4# | 5.5±0.5# | 5.2±0.4 |
| (M:K=30:70) | | | | | | |
| Ginkgolide V group (M:K=70:30) | 5.4±0.5 | 5.9±0.3# | 6.7±0.5# | 6.1±0.6# | 5.4±0.5# | 5.1±0.6 |
| Ginkgolide VI group (M:K=50:50) | 4.8±0.4# | 5.4±0.5# | 6.2±0.4# | 5.5±0.5# | 4.7±0.5# | 4.5±0.5# |

| | | | | | | |
|---|---|---|---|---|---|---|
| Note: compared with the single-use group (the GK group) with a better effect, #P is less than 0.05. | | | | | | |

As shown in Table 4, in this test example, compared with the GK group with a better effect, the ginkgolide IV group and the ginkgolide V group have lower clinical scores in D16-D19, the ginkgolide VI group has lower clinical scores in D14-D20, and differences have statistical significance. This shows that ginkgolide M and ginkgolide K in combined use have a synergistic effect.

**Table 5 Synergistic effect of ginkgolide B and bilobalide in combined use**

| Group | Clinical score | | | | | | | | | |
|---|---|---|---|---|---|---|---|---|---|---|
| | D12 | D14 | D16 | D17 | D18 | D19 | D20 | D22 | D24 | D26 |
| Ginkgolide B group | 3.4±0.5 | 3.8±0.6 | 4.5±0.5 | 5.1±0.6 | 4.8±0.4 | 4.2±0.4 | 3.9±0.6 | 3.3±0.5 | 2.9±0.7 | 2.4±0.5 |
| Bilobalide group | 3.6±0.5 | 3.9±0.6 | 4.6±0.5 | 5.2±0.6 | 4.9±0.6 | 4.3±0.5 | 4.0±0.7 | 3.4±0.5 | 2.9±0.7 | 2.4±0.5 |
| Ginkgolide 2 VII group (GB:BB =40:60) | 2.9±0.3# | 3.2±0.4# | 3.9±0.3# | | | 4.3±0.5#4.2±0.4#3.6±0.5#3.3±0.5#2.9±0.3# | | | 2.4±0.5 | 2.1±0.3 |
| Ginkgolide VIII group (GB:BB =65:35) | 2.8±0.4# | 3.1±0.3# | 3.8±0.4# | 4.1±0.6# | 4.0±0.5# | 3.5±0.5# | 3.1±0.6# | 2.8±0.4# | 2.3±0.5# | 1.9±0.3# |
| Ginkgolide IX group (GB:BB =50:50) | 2.7±0.5# | 3.0±0.0# | 3.3±0.5# | 3.5±0.5# | 3.5±0.5# | 2.9±0.3# | 2.8±0.4# | 2.7±0.5# | 2.3±0.5# | 1.9±0.3# |
| Ginkgolide X group (GA:GB:GC =1:1:1) | 3.1±0.6# | 3.4±0.5# | 4.1±0.6# | 4.5±0.5# | 4.3±0.5# | 3.7±0.5# | 3.3±0.5# | 2.9±0.3# | 2.4±0.5# | 2.1±0.3# |
| Ginkgolide XI group (A:B:C:BB =12:34:6:48) | 2.5±0.5# | 3.0±0.0# | 3.4±0.5# | 3.6±0.7# | 3.5±0.5# | 3.0±0.5# | 2.6±0.5# | 2.5±0.5# | 2.2±0.4# | 1.7±0.5# |

| | | | | | | | | | | |
|---|---|---|---|---|---|---|---|---|---|---|
| Note: compared with the single-use group (the GB group) with a best effect, #P is less than 0.05. | | | | | | | | | | |

As shown in Table 5, in this test example, compared with the GB group with the best effect in monomeric administration, the ginkgolide VII group, the ginkgolide VIII group, the ginkgolide IX group, the ginkgolide X group and the ginkgolide XI group have lower clinical scores in different days within a range of D12-D26. This shows that ginkgolide B and bilobalide in combined use have a synergistic effect, and ginkgolide A, ginkgolide B, ginkgolide C and ginkgo bilobalide in combined use have a synergistic effect.

**Table 6 Impact of ginkgolides on pain reactions of EAN model rats**

| Group | Pain reaction score (D18) |
|---|---|
| Negative control | 4.6±0.52^{∗} |
| Model control | 0.6±0.52 |
| Ginkgolide A group | 2.0±0.67^{∗} |
| Ginkgolide B group | 3.0±0.67^{∗} |
| Ginkgolide C group | 1.9±0.88^{∗} |
| Ginkgolide J group | 1.3±0.67^{∗} |
| Ginkgolide M group | 1.4±0.52^{∗} |
| Ginkgolide K group | 1.2±0.63^{∗} |
| Bilobalide group | 2.9±0.74^{∗} |
| Ginkgolide I group (A:C=40:60) | 2.5±0.53^{∗} |
| Ginkgolide II group (A:C=75:25) | 2.4±0.84^{∗} |
| Ginkgolide III group (A:C=50:50) | 2.7±0.67^{∗}# |
| Ginkgolide IV group (M:K=30:70) | 1.6±0.52^{∗} |
| Ginkgolide V group (M:K=60:40) | 1.7±0.80^{∗} |
| Ginkgolide VI group (M:K=50:50) | 2.2±0.42^{∗}$ |
| Ginkgolide VII group (GB:BB=40:60) | 3.3±0.67^{∗} |
| Ginkgolide VIII group (GB:BB=65:35) | 3.4±0.70^{∗} |
| Ginkgolide IX group (GB:BB=50:50) | 3.7±0.67^{∗}& |
| Ginkgolide X group (GA:GB:GC=1:1:1) | 3.6±0.52^{∗}& |
| Ginkgolide XI group (A:B:C:BB=12:34:6:48) | 3.8±0.79^{∗}& |

| | |
|---|---|
| Note: compared with the model control group, ^{∗}P is less than 0.05; compared with the GA group, #P is less than 0.05; compared with the GK group, $P is less than 0.05; and compared with the GB group, &P is less than 0.05. | |

As shown in Table 6, compared with the model group, the foregoing medicine administration groups have remarkably increased pain scores in D18, and differences have statistical significance. This shows that all the medicine administration groups of ginkgo terpene lactone can improve pain symptoms of EAN rats. In this test example, compared with the GA group or the GC group, the ginkgolide I group, the ginkgolide II group and the ginkgolide III group have increased scores, and differences in the ginkgolide III group have statistical significance. This shows that the ginkgolide III group has the best synergistic effect. Compared with the GM group or the GK group, the ginkgolide IV group, the ginkgolide V group and the ginkgolide VI group have increased scores, and differences in the ginkgolide VI group have statistical significance. This shows that the ginkgolide VI group has the best synergistic effect. Compared with the GB group or the BB group, the ginkgolide VII group, the ginkgolide VIII group, the ginkgolide IX group, the ginkgolide X group and the ginkgolide XI group have increased scores, and differences in the ginkgolide IX group, the ginkgolide X group and the ginkgolide XI group have statistical significance. The ginkgolide VIII group has the best synergistic effect, the ginkgolide X group in combined use has a synergistic effect, and the ginkgolide XI group in combined use has a synergistic effect.

### 3. Conclusion

In conclusion, the results of clinical scores and pain scores show that, ginkgolide A, ginkgolide B, ginkgolide C, ginkgolide J, ginkgolide M, ginkgolide K and bilobalide in single use, ginkgolide A and ginkgolide C in combined use, ginkgolide M and ginkgolide K in combined use, ginkgolide B and bilobalide in combined use, ginkgolide A, ginkgolide B and ginkgolide C in combined use, and ginkgolide A, ginkgolide B, ginkgolide C and bilobalide in combined use can improve clinical scores and pain scores of EAN rats, and all the medicines in combined use have a synergistic effect.

### Test example 2 Treatment effect of ginkgo terpene lactone on experimental autoimmune neuritis (EAN) model rabbits

### 1. Materials and methods

### 1.1 Experimental animals

210 half male and half female big-ear white rabbits, with a weight of 2.0-2.4 kg, and purchased from Sichuan Laboratory Animal Special Committee Farm.

### 1.2 Reagents and medicines

Complete Freund's adjuvant, Sigma-Aldrich. Bovine sciatic nerve myelin basic protein (P2), and experimental rabbit immune globulin. PCR primers and internal reference B-actin (GAPDH), Trizol reagent, RT-PCR kit (Dalian Takara Company); PCR Marker (American Promega Corporation).

### 1.3 Grouping and Modeling

21 experimental groups, 8 rabbits in each group.
Negative control group: normal saline
Model control group: normal saline
Positive control group: rabbit immune globulin, 400 mg/kg.d, intraperitoneal injection, for 5 consecutive days

The ginkgolide groups are set the same as Test example 1, i.e., include the ginkgolide A group to the ginkgolide K group (GA group to GK group), the bilobalide group (BB group) and the ginkgolide I group to the ginkgolide XI group, 18 groups in total. Ginkgolide used in each group has the same monomeric composition and ratio as those of Test example 1, but only differs in that the ginkgolide groups all use the same medicine administration dosage, i.e., 4.5 mg/kg.

Intragastric administration, 2 times a day. Medicines were dissolved in accessories (glycerin:ethanol=6:4 V/V) to reach 5 mg/ml, and then were configured to reach corresponding concentrations by using sterilized drinking water.

EAN rabbit modeling: a certain amount of bovine sciatic nerve myelin basic protein and an isopyknic complete Freund's adjuvant were taken, shaken up by using a test tube oscillator, sufficiently mixed, and then subcutaneously injected into a back of each rabbit at multiple points.

Clinical manifestations of the rabbits started to be observed every day from the second day after immunization, to determine a clinical process of the rabbits. Modeling succeeds when the following symptom manifestations occur: listlessness, less movements, anorexia, weight loss, unclean fur on tails and feet, weakness of limbs, flaccid paralysis, weight on hind legs, and slower reflection and stress function.
1.4 mRNAlevels of sciatic nerve tissue Th cells TNF-α, IL-4, IL-10 and IL-12

Anesthesia was performed on the 14th day after modeling, to take sciatic nerve tissue of rabbits, and total RNA was extracted by a Trizol method. Detection was performed by a RT-PCR method. (GBS is an autoimmune disease of a nervous system. In-vivo autoreactive T cells of a patient have a cellular immunologic response to myelin P2 antigens, to form a delayed allergic reaction peripheral nerve demyelination pathological injury. This process is mainly mediated by CD4⁺T cells, also named initial Th cells that may be differentiated into Th1 and Th2 after antigenic stimulation. Th1 cytokines (represented by TNF-α and IL-12) mainly enhance a cell mediated immune response, and are inflammatory cytokines; and Th2 cytokines (represented by IL-4 and IL-10) mainly participate in an antibody mediated immune response, and have an immune down-regulation and anti-inflammation effect. In GBS and ENA research, unbalance of Th1/Th2 plays an important role in disease injuries.)

### 1.5 Mathematical statistics

Data is represented by mean±standard deviation (x̅±SD). Differences between two groups were compared. When P is less than 0.05, a statistic difference occurs.

### 2. Experimental results

**Table 7 Impact of ginkgo terpene lactone on Th cytokines of EAN model rabbits**

| Group | Cytokines | | | |
|---|---|---|---|---|
| | TNF-α | IL-12 | IL-4 | IL-10 |
| Negative control | 1.023±0.044^{∗} | 1.456±0.301^{∗} | 0.677±0.070^{∗} | 0.823±0.045^{∗} |
| Model control | 5.009±0.171 | 6.637±0.232 | 0.479±0.106 | 0.470±0.013^{∗} |
| Positive control | 1.325±0.093^{∗} | 1.641±0.241^{∗} | 1.018±0.100^{∗} | 1.235±0.137^{∗} |
| Ginkgolide A group | 4.693±0.115^{∗} | 5.977±0.141^{∗} | 0.569±0.048^{∗} | 0.584±0.039^{∗} |
| Ginkgolide B group | 2.978±0.102^{∗} | 3.928±0.409^{∗} | 0.769±0.041^{∗} | 0.813±0.055^{∗} |
| Ginkgolide C group | 4.548±0.154^{∗} | 6.009±0.357^{∗} | 0.585±0.053^{∗} | 0.561±0.090^{∗} |
| Ginkgolide J group | 4.810±0.070^{∗} | 6.211±0.489^{∗} | 0.551±0.050^{∗} | 0.501±0.037^{∗} |
| Ginkgolide M group | 4.797±0.147^{∗} | 6.260±0.411^{∗} | 0.558±0.050^{∗} | 0.550±0.061^{∗} |
| Ginkgolide K group | 4.739±0.094^{∗} | 6.412±0.169^{∗} | 0.557±0.046^{∗} | 0.524±0.026^{∗} |
| Bilobalide group | 3.054±0.182^{∗} | 4.087±0.275^{∗} | 0.792±0.065^{∗} | 0.783±0.128^{∗} |
| Ginkgolide I group (A:C=40:60) | 4.320±0.198^{∗}# | 5.671±0.283^{∗}# | 0.644±0.042^{∗}# | 0.661±0.072^{∗}# |
| Ginkgolide II group (A:C=75:25) | 4.294±0.167^{∗}# | 5.725±0.191^{∗}# | 0.648±0.039^{∗}# | 0.681±0.053^{∗}# |
| Ginkgolide III group (A:C=50:50) | 4.060±0.141^{∗}# | 5.308±0.180^{∗}# | 0.685±0.040^{∗}# | 0.714±0.065^{∗}# |
| Ginkgolide IV group (M:K=30:70) | 1.531±0.232^{∗}& | 5.724±0.159^{∗}& | 0.610±0.040^{∗}& | 0.623±0.058^{∗}& |
| Ginkgolide V group (M:K=60:40) | 1.509±0.266^{∗}& | 5.642±0.108^{∗}& | 0.606±0.054^{∗}& | 0.627±0.041^{∗}& |
| Ginkgolide VI group (M:K=50:50) | 1.386±0.147^{∗}& | 5.306±0.306^{∗}& | 0.637±0.044^{∗}& | 0.669±0.034^{∗}& |
| Ginkgolide VII group (GB:BB=40:60) | 2.762±0.235^{∗△} | 3.580±0.153^{∗△} | 0.849±0.029^{∗△} | 0.866±0.031^{∗△} |
| Ginkgolide VIII group (GB:BB=65:35) | 2.589±0.404^{∗△} | 3.532±0.099^{∗△} | 0.854±0.044^{∗△} | 0.871±0.035^{∗△} |
| Ginkgolide IX group (GB:BB=50:50) | 2.193±0.198^{∗△} | 3.287±0.175^{∗△} | 0.875±0.044^{∗△} | 0.908±0.073^{∗△} |
| Ginkgolide X group (GA:GB:GC=1:1:1) | 3.232±0.255^{∗} | 4.013±0.198^{∗} | 0.707±0.063^{∗} | 0.792±0.072^{∗} |
| Ginkgolide XI group (A:B:C:BB=12:34:6:48) | 1.844±0.166^{∗△} | 2.834±0.100^{∗△} | 0.958±0.057^{∗△} | 1.011±0.111^{∗Δ} |

| | | | | |
|---|---|---|---|---|
| Note: compared with the model control group, ^{∗}P is less than 0.05; compared with the ginkgolide A group or the ginkgolide C group, #P is less than 0.05; compared with the ginkgolide J group or the ginkgolide M group or the ginkgolide K group, &P is less than 0.05; and compared with the ginkgolide B group and the BB group, Δp is less than 0.05. | | | | |

Results are shown in Table 7. In this test example, compared with the model group, the ginkgo terpene lactone monomer compounds in single use and in combined use by twos, threes and fours can improve levels of Th cytokines, and differences have statistical significance. Th1 cytokines (represented by TNF-α and IL-12) mainly enhance a cell mediated immune response, and are inflammatory cytokines; and Th2 cytokines (represented by IL-4 and IL-10) mainly participate in an antibody mediated immune response, and have an immune down-regulation and anti-inflammation effect. This shows that the foregoing medicine administration groups can adjust or correct an unbalanced Th1/Th2 cytokine network and adjust the balance of endogenous cytokines, thereby controlling the process of a disease and achieving a treatment effect. Through the comparison of effects of all the groups, this further shows that ginkgolide A and ginkgolide C in combined use have a synergistic effect, ginkgolide M and ginkgolide K in combined use have a synergistic effect, ginkgolide B and bilobalide in combined use have a synergistic effect, ginkgolide A, ginkgolide B, ginkgolide C and bilobalide in combined use have a synergistic effect, and all monomers in the ginkgolide XI group in combined use have the best effect.

### Embodiment 1

Active ingredients: bilobalide 1000 g (1 part) ginkgolide B 1000 g (1 part)
Preparation method: To the above raw materials, 1 part of glycerol monostearate, 1 part of polyethylene glycol, 1 part of carrageenan, 1 part of Arabic gum and 1 part of hydroxypropyl methyl cellulose were added to be evenly mixed, the obtained mixture was added into a pill dropping machine with a water dissolution device to be evenly heated and stirred in due time, the mixture was molten and then dropped into a condensate that is not mixed with the mixture, dropping pills were taken out after condensation, and the condensate attached to the surfaces of the dropping pills was wiped off, and then the dropping pills were dried at low temperature.

### Embodiment 2

Active ingredients: bilobalide 1000 g (1 part) ginkgolide B 500 g (0.5 part) ginkgolide A 250 g (0.25 part) ginkgolide C 250 g (0.25 part)
Preparation method: To the above raw materials, 1 part of glycerol monostearate, 1 part of polyethylene glycol, 1 part of carrageenan, 1 part of Arabic gum and 1 part of hydroxypropyl methyl cellulose were added to be evenly mixed, the obtained mixture was added into a pill dropping machine with a water dissolution device to be evenly heated and stirred in due time, the mixture was molten and then dropped into a condensate that is not mixed with the mixture, dropping pills were taken out after condensation, and the condensate attached to the surfaces of the dropping pills was wiped off, and then the dropping pills were dried at low temperature.

### Embodiment 3

Active ingredients: bilobalide 1000 g (1 part)
Preparation method: To the above raw materials, 1 part of glycerol monostearate, 1 part of polyethylene glycol, 1 part of carrageenan, 1 part of Arabic gum and 1 part of hydroxypropyl methyl cellulose were added to be evenly mixed, the obtained mixture was added into a pill dropping machine with a water dissolution device to be evenly heated and stirred in due time, the mixture was molten and then dropped into a condensate that is not mixed with the mixture, dropping pills were taken out after condensation, and the condensate attached to the surfaces of the dropping pills was wiped off, and then the dropping pills were dried at low temperature.

### Embodiment 4

Active ingredients: bilobalide 520g (0.52 part) ginkgolide B 760 g (0.76 part) ginkgolide A 700 g (0.7 part) ginkgolide C 280 g (0.28 part)
Preparation method: To the above raw materials, 1 part of glycerol monostearate, 1 part of polyethylene glycol, 1 part of carrageenan, 1 part of Arabic gum and 1 part of hydroxypropyl methyl cellulose were added to be evenly mixed, the obtained mixture was added into a pill dropping machine with a water dissolution device to be evenly heated and stirred in due time, the mixture was molten and then dropped into a condensate that is not mixed with the mixture, dropping pills were taken out after condensation, and the condensate attached to the surfaces of the dropping pills were wiped off, and then the dropping pills were dried at low temperature.

### Embodiment 5

Active ingredients: bilobalide 960g (0.96 part) ginkgolide B 680 g (0.68 part) ginkgolide A 240 g (0.24 part) ginkgolide C 120 g (0.12 part)
Preparation method: To the above raw materials, 1 part of glycerol monostearate, 1 part of polyethylene glycol, 1 part of carrageenan, 1 part of Arabic gum and 1 part of hydroxypropyl methyl cellulose were added to be evenly mixed, the obtained mixture was added into a pill dropping machine with a water dissolution device to be evenly heated and stirred in due time, the mixture was molten and then dropped into a condensate that is not mixed with the mixture, dropping pills were taken out after condensation, and the condensate attached to the surfaces of the dropping pills was wiped off, and then the dropping pills were dried at low temperature.

### Test example 3 Observation of impact of resulting ginkgo terpene lactone preparations in Embodiment 1 to Embodiment 3 on treatment effect of GBS patients

### 1 Score standards

### 1.1 MRC scores

UK Medical Research Council (MRC) score standards were used to evaluate a sum of MRC scores of 6 muscle groups, including bilateral shoulder abductors, elbow flexors, extensor carpi, hip flexors, knee extensors and dorsi flexors. The MRC score of each muscle group ranges from 0 to 5, and the total score ranges from 0 (complete paralysis) to 60 (normal). Grades and scores of the muscle groups are as below:
0=No visible contraction;
1=Visible contraction but no movements;
2=Movable limbs but unable to resist gravity;
3=Able to resist gravity, substantially sufficient movement amplitude, but unable to resist resistance;
4=Able to resist gravity and resistance; and
5=Normal.

### 1.2 HFGS scores

Reference is made to Table 8.

**Table 8 HFGS score standards**

| Grade | Standard |
|---|---|
| 0 | Normal |
| 1 | Slight symptoms or signs, able to run |
| 2 | Able to walk by 5 m without help, but unable to run |
| 3 | Able to walk on flat ground by 5 m with help |
| 4 | Lie in bed or wheelchair-bound |
| 5 | Require assisted respiration |
| 6 | Death |

### 2. Experimental results

10 cases of GBS patients who took 160 mg/d of resulting dripping pills in Embodiment 1 for 30 days were observed. After comparison with MRC scores and HFGS scores of admitted patients, the total MRC score for treatment effect evaluation was raised by 2-8, and the HFGS grade was lowered by 1. No adverse reactions were observed.

9 cases of GBS patients who took 160 mg/d of resulting dripping pills in Embodiment 2 for 30 days were observed. After comparison with MRC scores and HFGS scores of admitted patients, the total MRC score for treatment effect evaluation was raised by 1-6, and the HFGS grade was lowered by 1. No adverse reactions were observed.

15 cases of GBS patients who took 160 mg/d of resulting dripping pills in Embodiment 3 for 30 days were observed. After comparison with MRC scores and HFGS scores of admitted patients, the total MRC score for treatment effect evaluation was raised by 3-7, and the HFGS grade was lowered by 1. No adverse reactions were observed.

### Test example 4 Observation of impact of resulting ginkgo terpene lactone preparations in Embodiment 4 and Embodiment 5 on treatment effect of GBS patients

A score standard that was the same as that of Test example 3 was used to observe treatment effects on GBS patients, and results were as below:
12 cases of GBS patients who took 160 mg/d of resulting dripping pills in Embodiment 4 for 30 days were observed. After comparison with MRC scores and HFGS scores of admitted patients, the total MRC score for treatment effect evaluation was raised by 3-8, and the HFGS grade was lowered by 1. No adverse reactions were observed.
17 cases of GBS patients who took 160 mg/d of resulting dripping pills in Embodiment 5 for 30 days were observed. After comparison with MRC scores and HFGS scores of admitted patients, the total MRC score for treatment effect evaluation was raised by 5-12, and the HFGS grade was lowered by 2. No adverse reactions were observed.

To sum up, the ginkgo terpene lactone can be clinically used to prevent and/or treat symptoms of Guillain-Barre-Strohl syndrome. An oral preparation of the ginkgo terpene lactone is convenient to take and has good patient compliance.

## Claims

1. Use of ginkgo terpene lactone or pharmaceutically-acceptable salts, esters, hydrates, solvates and isomers thereof, or any crystal form, racemate and metabolin thereof, or a mixture thereof in the manufacture of a medicament for preventing and/or treating Guillain-Barre-Strohl syndrome.

2. The use of claim 1, wherein the ginkgo terpene lactone is ginkgo sesquiterpene lactone or/and ginkgo diterpene lactone.

3. The use of claim 2, wherein the ginkgo terpene lactone is one or two or more of ginkgolide A, ginkgolide B, ginkgolide C, ginkgolide M, ginkgolide J, ginkgolide K, ginkgolide L, ginkgolide N, ginkgolide P, ginkgolide Q and bilobalide.

4. The use of claim 3, wherein the ginkgo terpene lactone is one combination selected from bilobalide:ginkgolide B=(35-60):(40-65) w/w; or ginkgolide A:ginkgolide C=(40-75):(25-60) w/w; or ginkgolide M:ginkgolide K=(30-60):(40-70) w/w; or ginkgolide A:ginkgolide B:ginkgolide C=1:1:1 (w/w/w); or ginkgolide A:ginkgolide B:ginkgolide C:bilobalide=12:34:6:48 (w/w/w/w).

5. The use of claim 3, wherein the ginkgo terpene lactone is a combination of ginkgolide A:ginkgolide B:ginkgolide C:bilobalide=(10-35):(20-38):(5-14):(26-50) w/w/w/w.

6. The use of claim 3, wherein the ginkgo terpene lactone is a combination selected from bilobalide:ginkgolide B=50:50 (w/w); or ginkgolide A:ginkgolide C=50:50 (w/w); or ginkgolide M:ginkgolide K=50:50 (w/w).

7. The use of any one of claims 1 to 6, wherein disease subtypes of the Guillain-Barre-Strohl syndrome comprise acute inflammatory demyelinating polyneuropathy, acute motor axonal neuropathy, acute motor sensory axonal neuropathy, Miller Fisher syndrome, acute panautonomic neuropathy and acute sensory neuropathy.

8. A medicament for preventing and/or treating Guillain-Barre-Strohl syndrome of any one of claims 1 to 6, wherein the medicament comprises ginkgo terpene lactone as an active ingredient, and a pharmaceutically-acceptable carrier, and the ginkgo terpene lactone is one combination selected from bilobalide:ginkgolide B=(35-60):(40-65) w/w; or ginkgolide A:ginkgolide C=(40-75):(25-60) w/w; or ginkgolide M:ginkgolide K=(30-60):(40-70)w/w ; or ginkgolide A:ginkgolide B:ginkgolide C=1:1:1 (w/w/w); or ginkgolide A:ginkgolide B:ginkgolide C:bilobalide=12:34:6:48 (w/w/w/w).

9. The medicament of claim 8, wherein the carrier comprises one or more selected from a filler, a diluent, a lubricant, a flow aid, an anti-adherent, a dispersing agent, a wetting agent, a binder, a modifier, a solubilizer, an antioxidant, a bacteriostat, an emulsifier and a disintegrant; wherein the binder comprises one or more selected from Arabic gum, gelatin, sorbitol, tragacanth gum, cellulose, microcrystalline cellulose, sodium carboxymethyl cellulose, ethyl cellulose, hydroxy propyl methyl cellulose, syrup, starch slurry and polyvinylpyrrolidone; wherein the filler comprises one or more selected from lactose, powdered sugar, dextrin, starch and a derivative thereof, cellulose and a derivative thereof, an inorganic calcium salt, sorbitol and glycine; wherein the lubricant comprises one or more selected from micronized silica gel, magnesium stearate, talcum powder, aluminum hydroxide, boric acid, hydrogenated vegetable oil and polyethylene glycol; wherein the disintegrant comprises one or more selected from starch and a derivative thereof, polyvinylpyrrolidone and microcrystalline cellulose; wherein the wetting agent comprises one or more selected from sodium lauryl sulfate, water and alcohol; wherein the antioxidant comprises one or more selected from sodium sulfite, sodium hydrogen sulfite, sodium metabisulfite and dibutyl benzoic acid; wherein the modifier comprises one or more selected from hydrochloric acid, citric acid, potassium hydroxide, sodium citrate and a buffer agent; wherein the emulsifier comprises one or more selected from polysorbate-80, fatty acid sorbitan, Pluronic F-68, lecithin and soybean lecithin; and wherein the solubilizer comprises one or more selected from Tween-80, bile and glycerin.

10. A method for preventing and/or treating Guillain-Barre-Strohl syndrome, comprising administering a therapeutically effective amount of ginkgo terpene lactone to a subject, wherein the ginkgo terpene lactone is as defined in any one of claims 1 to 6.
